# EUROPEAN PATENT SPECIFICATION

(11) **EP 2 764 890 B1**
(45) Date of publication and mention of the grant of the patent: **31.08.2016**
(21) Application number: 14153810.8
(22) Date of filing: 04.02.2014
(51) Int. Cl.: A61N 1/372, A61N 1/375, A61N 1/378, A61N 1/37

(54) **Implantable medical device, medical system and method for data communication**
Implantierbare medizinische Vorrichtung, medizinisches System und Verfahren zur Datenkommunikation
Dispositif médical implantable, système médical et procédé de communication de données

(30) Priority: 07.02.2013 US 201361761707 P; 21.11.2013 US 201361906902 P
(43) Date of publication of application: 13.08.2014
(73) Proprietor: BIOTRONIK SE & Co. KG, 12359 Berlin (DE)
(72) Inventor: Moulder, J. Christopher, Portland, OR Oregon 97202 (US)
(74) Representative: Galander, Marcus

(56) References cited:
- US-A1- 2008 294 062
- US-A1- 2009 228 071
- US-A1- 2010 125 315
- US-A1- 2011 125 214
- US-A1- 2011 196 452
- US-A1- 2011 288 615
- US-A1- 2012 004 708
- US-A1- 2012 029 323
- US-A1- 2012 101 545

## Description

The present invention generally relates to implantable medical devices and data communication from such implantable medical device to an external device.

Implantable devices, in particular implantable medical devices, such as implantable therapy and/or monitoring devices including pacemakers, cardioverters and defibrillators or the like, may include data communication means to transmit data from the implantable medical device to a (body-)external device or vice versa. The data communication means can be used when the implantable medical device is implanted in the body of a mammal or when it is stored in a conductive package prior to the implantation.

A system for data communication with a medical device thus includes an implantable medical device and an external device such as a programmer or communication device.

A typical implantable medical device comprises a battery, a monitoring and/or therapy control unit, in some cases one or more therapy units such as stimulation units and a memory for storing control program and/or data sensed by the implantable medical device. If the implantable medical device is a pacemaker or an implantable cardiovert-er/defibrillator (ICD), the therapy units comprise stimulation (pacing) units for generating and delivering electric stimulation pulses to a patient's heart tissue (myocardium). Often, sensing units for sensing cardiac activity are provided. Sensing units can process electrical signals that represent electrical potentials that can be picked up via electrodes, e.g., in a heart.

In order to transmit data stored by the implantable medical device to an external device a telemetry unit may be provided. Typically the telemetry unit is configured to allow a bidirectional data communication, that is, the telemetry unit can transmit and receive data wirelessly.

US2008294062A1 discloses an ultra-low-power circuit for wireless neural recording and stimulation, wherein the circuit includes a neural amplifier with adaptive power biasing for use in multi-electrode arrays and a decoding and/or learning architecture.

US2009228071A1 describes techniques for communication between at least one lead-borne device of an implantable lead and a medical device to which the lead is connected.

US2012004708A1 discloses an implantable medical device and an external base station system, wherein the external base station can provide a passive electric field to power the implant, or to charge its battery.

US2012029323A1 discloses an antenna for an implantable medical device for wirelessly communicating with another device.

US2010125315A1 discloses a method and system of providing therapy to a patient implanted with an array of electrodes.

Limited battery capacity of an implantable medical device calls for energy-efficient data communication. An implantable medical device with limited battery power requires a low power communication scheme in order to program it and to download acquired data. With extremely low power communication, device longevity is increased and longer communication sessions may be achieved.

Present communication schemes or data communication by a telemetry unit may involve RF, magnetic, ultrasonic or galvanic communication. RF frequencies of ∼400 or ∼900 MHz or magnetic coupling in the 100s of kHz range require several mA of current to transmit and receive data. Such high current requirements are out of reach of devices with battery capacities of at most a few hundred mAh. Active transmission of galvanic pulses may also require several mA of current.

In addition, RF schemes require relatively large antennas and magnetic coupling requires large transmit and receive coils for communication. These transmission schemes also do not penetrate the body effectively and have ranges on the order of 5 - 10 cm. The space available in an implantable leadless pacer (iLP), for instance, would not allow such large coils or antennas. iLPs are designed to be placed within a heart chamber as opposite to conventional pacemakers, where the pacemaker itself is placed outside the heart and electrode leads extend from the pacemaker into the heart.

In view of the above, there is a need for a low-power communication scheme that does not employ RF or magnetic coupling.

It is an object of the invention to provide an implantable medical device with a data communication method that causes minimal battery drain from the medical device's battery. The data communication method may be used while the implantable medical device is sensing physiological signals. An implantable medical device is provided that comprises data communication means which include means for altering an oscillatory electric field imposed on a conductive medium surrounding the implantable medical device. The conductive medium is either the body tissue when the implantable medical device is implanted in the body of a mammal or a conductive package in which the implantable medical device is stored prior to the implantation.

The present application discloses modulation of impedance surrounding the implantable medical device within an electric field to communicate between a small implantable medical device implanted in the heart or body or stored in a conductive package and an external device like a programmer or communication device. Alternatively, the implantable medical device may generate a small electric field to facilitate this same communication. In one embodiment the external device uses surface electrodes to impart an oscillatory electric field on the body or package that encompasses the implantable medical device and a second set of electrodes to sense the impedance change.

In a first embodiment, the means for altering an oscillatory electric field are configured to modulate a impedance surrounding the implantable medical device when the implantable medical device is within a conductive medium within an oscillatory electric field. The external device uses surface electrodes to impart an oscillatory electric field that encompasses the implantable medical device. The oscillatory electric field can have an oscillation frequency in the range of 50 kHz to 1 MHz and preferably has an oscillation frequency in the range of 400 - 700 kHz. The implantable medical device, within the field, alternates shorting and opening an internal connection between two electrodes on its surface to change the impedance across the space of the implantable medical device. The change in impedance is sensed by the external device as changes in either current or voltage of the imparted oscillatory field in the vicinity of the implantable medical device. A lock-in amplifier is used to average the small voltage or current change over several cycles of the injected field. The changes in voltage or current can be modulated to form a communication scheme to transmit data.

In an alternative embodiment, the means for altering an oscillatory electric field comprise generating an oscillatory electric field that is phase-synchronized with an oscillatory electric field imposed on the implantable medical device when the implantable medical device is surrounded by a conductive medium. The external device uses surface electrodes to impart an oscillatory electric field that encompasses the implantable medical device. The implantable medical device then monitors this field and generates a small electric field which is phase-synchronized with the external devices field in order to facilitate reception of this small field by the external device using a lock-in amplifier. The small changes in the electric field are sensed by the external device as changes in voltage or current of field surrounding the implantable medical device. The changes in voltage or current are demodulated to form a communications scheme from the implantable medical device to the external device. Synchronization with the external field allows the implantable device to generate a much smaller electric field than would otherwise be required to communicate with the external device.

In another alternative embodiment, the implantable medical device generates a small oscillatory field at a predetermined frequency between its electrodes. The external device senses this frequency and phase-locks to it in order to facilitate reception of the small field using a lock-in amplifier. The oscillatory field generated by the implantable medical device is on the order of 1 mV - 50 mV and preferably around 30 mV. The changes in oscillatory are demodulated to form a communications scheme from the implantable medical device to the external device. Synchronization with the external field allows the implantable device to generate a much smaller electric field than would otherwise be required to communicate with the external device. By generating an oscillatory field and sensing the field using a lock-in amplifier, the embodiment may also be extended to communication between two implantable medical devices.

Because very little power is required for data transmission, an implantable medical device with limited battery supply has the ability to transmit increased amount of data while using very little power. Data transmission is achieved by modulating an electric field surrounding the device and read by the receiver as changing voltage or current. Continuous medium rate data transmission can be achieved while using very little battery power.

Preferably, the implantable medical device according to the first embodiment further comprises a switch, such as a FET, relay, BJT, or similar switching device, and at least two electrodes that are connected to the switch so they can be electrically connected or disconnected, respectively, by the switch, wherein the at least two electrodes are configured and arranged to cause a change of impedance when the implantable medical device is surrounded by a conductive medium, such as body tissue or a conductive package, and the electrodes are connected or disconnected, respectively, by means of the switch, e.g., a switch or in particular a low impedance solid state switch.

In one embodiment, one electrode, a tip electrode, is arranged at the tip of the implantable medical device. Another electrode, a ring electrode, can be arranged on or around the surface of the implantable medical device. The tip and ring electrodes can be used to pace a heart by inducing electrical cardiac activity (pacing), to sense the electrical activity of a heart (sensing), and/or to alter the local electric field for data communication to transmit data to an external device (transmitting). A preferred frequency for data communication is 4 - 8 kHz, but suitable a range would also be 1 - 20 kHz. The implantable medical device can also include more electrodes, e.g., a pair of electrodes for each of the tasks (pacing, sensing, and transmitting) of the implantable medical device.

The switch may be connected to a switch control that is configured to sense an oscillatory electric field imposed on the implantable medical device. Thus it is possible to synchronize connecting and disconnecting of the at least two implantable electrodes with the oscillatory electric field imposed on conductive medium surrounding or encompassing the implantable medical device.

To implement such synchronizing, the switch control may comprise a phase-locked loop (PLL) and a frequency divider, wherein the phase-locked loop is configured to lock in a frequency of an oscillatory electric field imposed on body tissue surrounding the implantable medical device when the implantable medical device is in its implanted state. The PLL is desirable when fast transmitting speeds are required, thus reducing the number of cycles of the external oscillatory field when the switch is active in order to decode the modulated carrier signal at the external device. The frequency divider is connected to the phase-locked loop and is configured to divide a frequency signal put out by the phase-locked loop. Thus, the implantable medical device can generate a code that represents data to be transmitted from the implantable medical device to an external device, wherein the clock for such code is a fraction of the frequency of the oscillatory electric field imposed on the body tissue surrounding the implantable medical device. The clock frequency is preferably in a range of 0.1 to 50 kHz, such as a range of 1 to 20 kHz, more preferably in a range of 4 to 10 kHz, most preferably the clock frequency is 8 kHz.

According to a further embodiment, the switch control is connected to the at least two electrodes and is configured to sense an oscillatory electric field imposed on the at least two electrodes. The switch control can comprise a band-pass filter, wherein the band-pass filter filters a signal fed to the phase-locked loop.

In a preferred embodiment of the implantable medical device no PLL is required and the switching of the electrodes is not synchronized to the external oscillating field. Because the external oscillating field is at a much higher frequency than the frequency of switching within the device, for example approximately 100:1, the lock-in amplifier is able to adequately amplify the received signal.

In an alternative embodiment, the means for altering an oscillatory electric field comprise field generating means for generating an oscillatory electric field that is phase-synchronized with an oscillatory electric field imposed on a conductive medium surrounding the implantable medical device. An implantable medical device according to this embodiment may further comprise field generation control means that are operatively connected to the field generating means and are configured to control the field generating means in response to an oscillatory electric field imposed on a conductive medium surrounding the implantable medical device.

The implantable medical device can further include means for sensing electrograms or can be configured to sense electrograms. Preferably the implantable medical device is configured to allow simultaneous communication between the implantable medical device and an external device and sensing electrograms using the implantable medical device.

The implantable medical device preferably includes pace/sense circuitry comprising a DC blocking capacitor and input amplifiers for impedance measurements and capture control. Input amplifiers for impedance measurements and capture control can become saturated after pacing pulses are delivered to a heart. The excess energy is then deposited on the DC blocking capacitor and removed by shorting the DC blocking capacitor across the electrodes, which causes the input amplifier to saturate. The electrodes of the implanted medical device create a slight half-cell potential between the metal of the electrodes and the blood electrolyte. This potential is a DC voltage that is normally filtered by the input amplifier and does not affect sensing. If the electrodes are shorted together, as may occur during modulation of the oscillatory field, there is an instantaneous change in the potential that requires several milliseconds to restabilize. The time constant is dependent on the tissue impedance. The instantaneous change in potential causes the input amplifier to saturate, during which time no electrogram can be sensed.

In a preferred embodiment of the implantable medical device a means for maintaining any DC potential and other low frequency signals across the device electrodes, such as a capacitor, is placed in series with the switching means or the switch of the switching means and between the two electrodes that can be connected and/or disconnected by the switching means to cause a change of impedance, said capacitor serves to maintain a DC voltage on the electrodes when they are connected by the switching means. By maintaining the DC voltage on the electrodes, the implantable medical device can sense electrical cardiac activity and simultaneously communicate with an external device. It is thus not required to limit the data communication to "safe" windows when sensing is not required.

Preferably the switch causes low charge injection and is low impedance. Charge injection by the switch can cause slight noise on the electrogram sensing. There is a trade-off between charge injection and switch impedance. If charge injection by the switch is high, the charge will be deposited on the capacitor and the electrodes and cause switching artifacts. An electrogram can nonetheless be acquired but high frequency noise can be present. Switch impedance affects the amplitude of the reflected signal to the external device. The higher the impedance the lower the reflected signal. The capacitor preferably has low impedance to high frequency, e.g., 1 Ohm and high impedance to DC and low frequency, preferably larger than 2500 Ohms. Shorting together the electrodes by the switch preferably shorts a high frequency carrier signal through the capacitor and alters the local high frequency electric field, which effects communication. Low frequency physiologic signal and DC offset voltage on the electrodes is preferably not affected and maintained by the capacitor. Additionally an additional resistor can be placed in parallel with the switch of the switch and in series with the capacitor in order to maintain DC voltage on the capacitor when communication is not active.

A preferred embodiment of the implantable medical device with an electrogram amplifier front-end includes an electrogram amplifier, an input resistor, and a filter resistor in combination with a filter capacitor, which form a low-pass filter at the electrogram amplifier front-end. When the switch is closed, the input resistor is shorted to low impedance and most of the input voltage falls across the filter capacitor. The filter capacitor can also act to maintain the DC potential on the electrodes because it is continually charged by the filter resistor bypassing the switch. When the switch is open the filter resistor and filter capacitor act as a low-pass filter, e.g., with 39 kOhms and 33 nF the low-pass filter has a cutoff frequency of about 124 Hz. The filter is intended to be used to prevent high frequency noise from entering the amplifier. When the switch is closed the filter cutoff frequency is shifted to a higher frequency in dependence on the switch impedance, e.g., preferably slightly below the carrier frequency, while electrogram acquisition is maintained. The high frequency carrier signal used for conductive intra-body communications/impedance-communication can be shunted through the capacitor to the other electrode. Shunting of the high frequency alters the local electric field around the implantable medical device allowing communication.

The objective is further achieved by a data communication system including an implantable medical device as described above and an external device that comprises or can be connected to at least two surface electrodes. The external device further comprises external field generating means, such as a current or voltage source, that are configured to generate an oscillatory electric field to be imposed on a body or other conductive medium such as a package via the at least two surface electrodes. The external device further comprises means for sensing alterations of body or package impedance and/or an oscillatory electric field generated by the implantable medical device.

The external device preferably comprises a lock-in amplifier, an AM demodulator for demodulating amplitude-modulated signals and an analog-to-digital converter, wherein the analog-to-digital converter is operatively connected to the AM demodulator and the lock-in amplifier, and wherein the analog-to-digital converter puts out a signal that represents a signal transmitted by the implantable medical device. The preferred embodiment further comprises four electrodes, two for injecting the field and two to receive the modulated signal from the implantable medical device.

In one embodiment the external device is configured to receive alterations of impedance that are modulated to encode data in a binary phase shift key method. The AM demodulated signal is encoded in such a manner that there are no long periods in which the implantable medical device switch is in any single position, either on or off. One example of such an encoding scheme is to encode a logical '1' as modulating the switch closed-open-closed-open and a logical '0' as modulating the switch open-closed-open-closed. In this manner the switch is either closed or open twice as long when transitioning from a '0' to '1' or '1' to '0', respectively. The fundamental frequency of switching is thus maintained and narrower band-pass filters may be used in the lock-in amplifier. This in turn increases signal-to-noise ration of the received communication from the implantable medical device.

In another embodiment the external device uses two lock-in demodulators phase shifted by 90 degrees. By demodulating the received signal at, for example 0 degrees and 90 degrees, the external device may maintain a high signal-to-noise ratio by combining the two signals. Phase information may also be decoded based on the difference in amplitude of the two output signals. Phase information can be used to implant more advanced modulating schemes in the implantable medical device. For example, the oscillatory field generated by the implantable medical device may be phase shifted such that the output at the external device is either positive or negative, depending on the phase output. In this manner a continuous oscillatory field may be realized where phase shifting segments encodes data in the communication. Further, phase discrimination may allow determination of the implantable device with respect to the receiving electrodes.

In a still further embodiment of the external device, only two electrodes are used to both impart the oscillatory field on a body or other conductive medium such as a package, and to receive the modulated signal from the implantable medical device. In this configuration a Wheatstone bridge may be used such that the imparted signal is between the surface electrodes and the received signal is between one surface electrode and an internal connection. Using two surface electrodes for the external device reduces external components and simplifies operation.

In a preferred embodiment the implantable device, i.e. implantable medical device, has a hermetically sealed housing. The hermetically sealed implantable device or implantable medical device with a hermetically sealed housing can be a medical therapy and/or monitoring device.

A method of communicating data from an implantable medical device to an external device is further described, said method comprising:
- imposing an oscillatory electric field on a conductive medium encompassing an implantable medical device,
- altering the oscillatory electric field by means of the implantable medical device
   - by inducing an alternating change of impedance by means of the implantable medical device or
   - by generating a small oscillatory electric field by means of the implantable medical device,
- sensing the change of impedance or the small oscillatory electric field generated by the implantable medical device, respectively, by means of an external device having or being connected to at least two surface electrodes.

The method may include the step of sensing the imposed oscillatory electric field by means of the implantable medical device after the step of imposing an oscillatory electric field on a conductive medium encompassing an implantable medical device. In this case the implantable medical device may communicate only if an oscillatory electric field is detected. Preferably, the step of altering the oscillatory electric field by means of the implantable medical device is performed by means of at least two electrodes operatively connected or being part of the implantable medical device, and switch operatively connected to the at least two electrodes, wherein the switch connects and disconnects the at least two electrodes in an alternating manner according to a code-representing data to be transmitted from the implantable medical device to the external device, said alternating connecting and disconnecting causing a detectable change of impedance for the imposed oscillatory electric field. Said switch has a high impedance at low frequency and low impedance at high frequency and is in series with a capacitor or other means to maintain a DC voltage on said electrodes.

The method of data communication can include data communication from an external device to an implantable device. Preferably the method comprises the step of modulating the oscillatory electric field by means of the external device.

The above and other aspects, features and advantages of the present invention will be more apparent from the following more particular description thereof, presented in conjunction with the following drawings, wherein:
- Figure 1:: is a representation of a communication system, comprising an implantable medical device in its implanted state and an external device;
- Figure 2:: is a more abstract representation of the system depicted in Fig. 1;
- Figure 3:: is an alternative embodiment of the system depicted in Fig. 1;
- Figure 4:: is a more detailed representation of another alternative embodiment of an implantable device showing those elements that are provided to implement the invention;
- Figure 5:: is a more detailed representation of an external device showing those elements that are provided to implement the invention;
- Figure 6:: is a representation of a configuration of a passive Z-COMM switch;
- Figure 7:: is a representation of a low-pass filter implementation of a switch circuit;
- Figure 8:: is a diagram illustrating the change of the frequency response and in particular the low-pass filter cutoff frequency (corner) change during transmission;
- Figure 9:: is a circuit diagram for a simulation scenario for simulating impedance change based data communication;
- Figure 10:: is a plot illustrating a simulation result achieved by using the diagram depicted in Fig. 9.

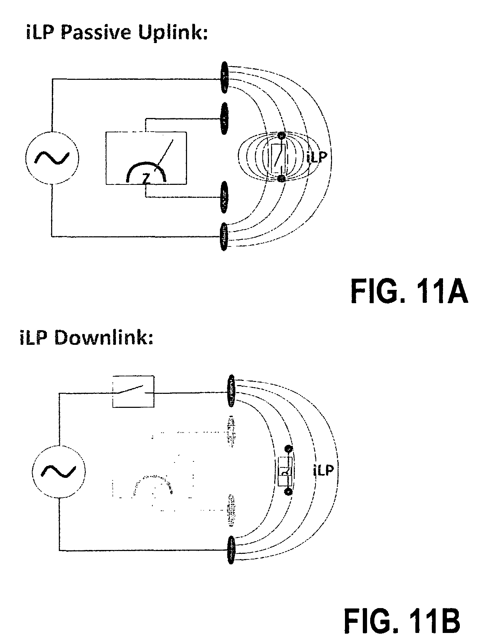

- Figure 11A:: is a representation of communication between the implantable medical device and an external device showing the imparted oscillatory field and the local oscillatory field being modulated by the implantable medical device.
- Figure 11B:: is a representation of communication between the external device and the implantable medical device.

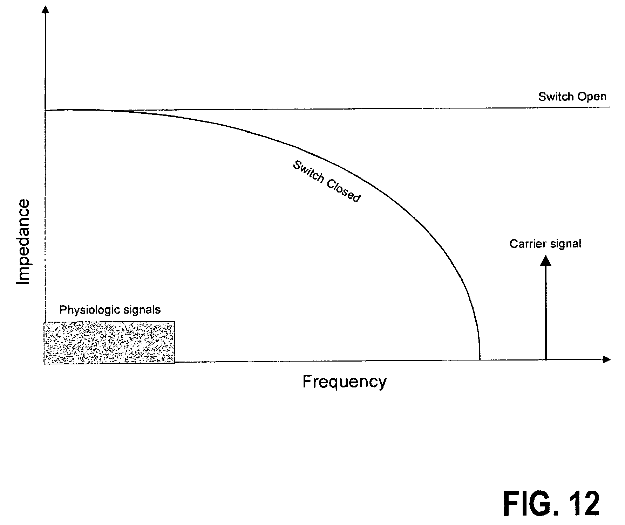

- Figure 12:: is diagram showing the change of impedance across the implantable medical device when the modulating switch is open can closed.

The following description is of the best mode presently contemplated for carrying out the invention. This description is not to be taken in a limiting sense, but is made merely for the purpose of describing the general principles of the invention. The scope of the invention should be determined with reference to the claims.

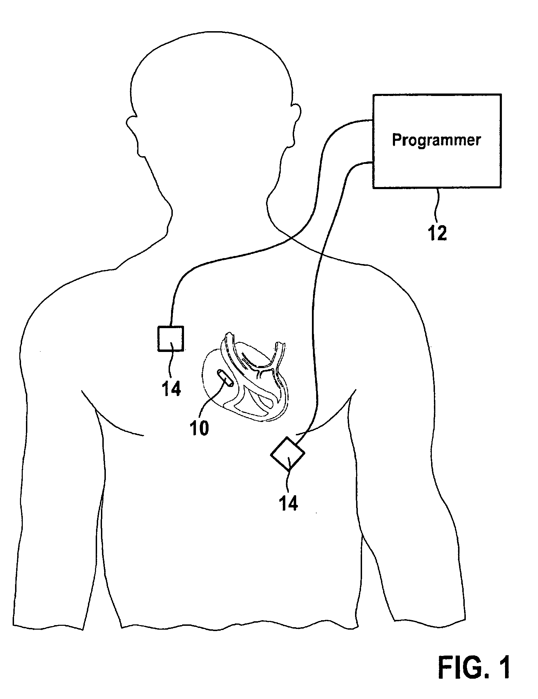

In one embodiment, utilizing an implanted pacemaker as an implantable medical device 10, a programmer or communication device as external device 12 uses surface electrodes 14 placed on either side of the heart as shown in Fig. 1. The external device 12 induces an oscillating electric field between the electrodes between 50 kHz to 1 MHz, preferably between 50 to 100 kHz, at a known voltage or current. The medical device implanted in the heart is located between the two surface electrodes 14.

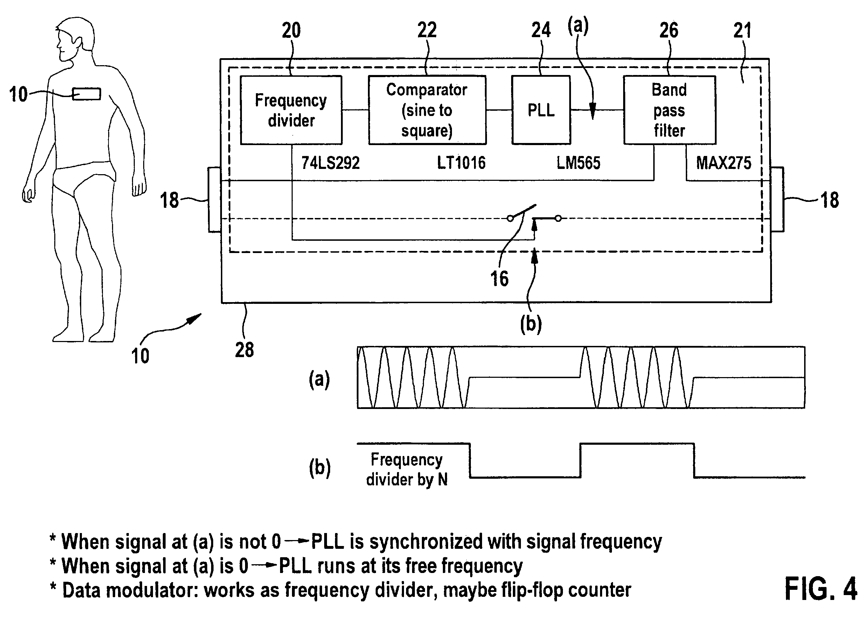

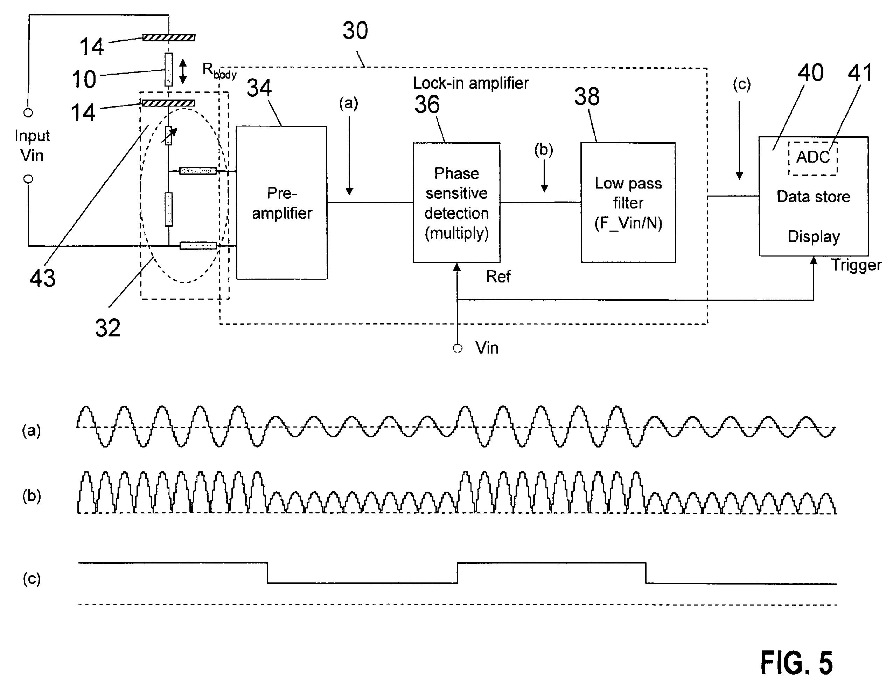

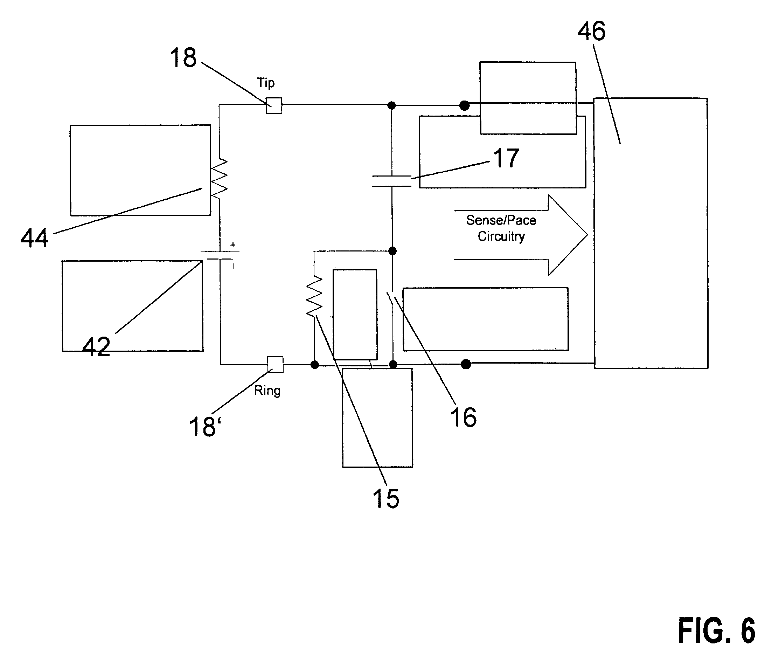

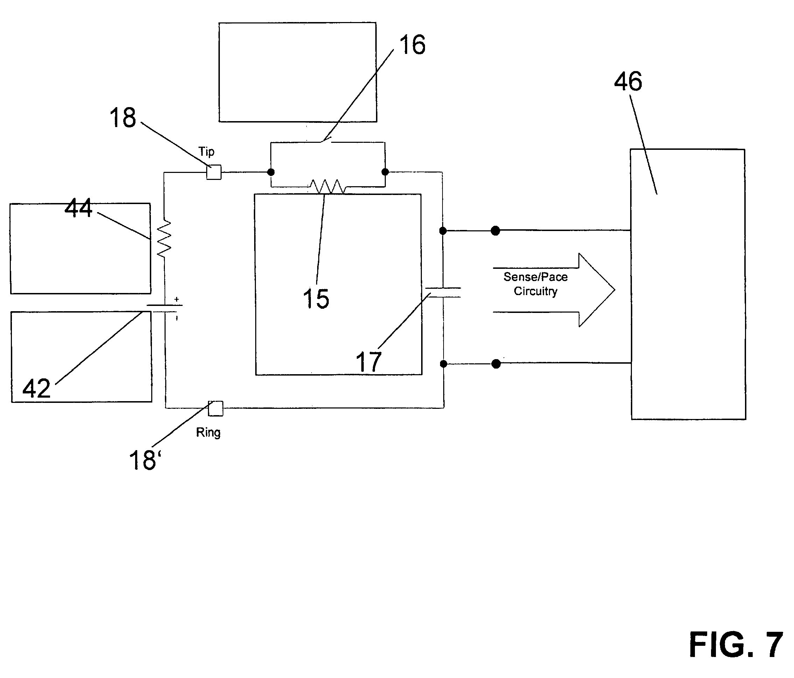

In the more detailed representations of the implantable medical device 10 and the external device 12 in Fig. 4 and 5, only those elements are depicted that are provided to implement the invention. It should be noted that a typical implantable medical device 10 such as an implantable heart stimulator may further comprise a battery, a monitoring and/or therapy control unit, one or more therapy units such as stimulation (pacing) units, sensing units or the like, pace/sense circuitry 46 shown in Fig. 6 and Fig. 7 and a memory for storing control program and/or data sensed by the implantable medical device. If the implantable medical device 10 is a pacemaker or an implantable cardioverter/defibrillator (ICD), the therapy units comprise stimulation units for generating and delivering electric stimulation pulses to a patient's heart tissue (myocardium). Stimulation units can be or are connected to stimulation electrode leads in a manner known per se.

In order to transmit data sensed by the implantable medical device 10 to an external device 12 a telemetry unit is provided. Typically the telemetry unit is configured to allow a bidirectional data communication, that is, the telemetry unit can transmit and receive data wirelessly. Figures 4 and 5 illustrate details of internal and external therapy units, respectively.

Referring to Fig. 4, the implantable medical device 10 comprises two (implantable) electrodes 18 that contact body tissue surrounding the implantable medical device 10 in its implanted state. Switching means represented by a simple switch 16 serve for connecting or disconnecting, respectively, the two electrodes 18 in order to establish or interrupt an electrical connection between the electrodes 18. The electrical connection across switching means 16 shall be of low ohmic resistance. The at least two electrodes 18 of the implantable medical device 10 preferably are arranged on the external surface of a hermetically sealed housing 28 encapsulating the implantable medical device 10. According to a preferred embodiment, parts of the housing 28 itself form the at least two electrodes 18. The electrodes 18 can also be formed by a tip electrode 18 located at a tip of the implantable medical device 10 and a ring electrode 18' located on the circumference of the implanted medical device 10 (not shown).
If the implantable medical device is connected to leads carrying electrodes in contact with body tissue, any two of these electrodes including the conductive housing can be used as electrodes 18.

In order to control switching means 16, the implantable medical device 10 may further comprise a frequency divider 20 connected to a sine-to-square converting comparator 22 that in turn is connected to a phase-locked loop (PLL) 24. Phase-locked loop 24 is connected to the electrodes 18 via band-pass filter 26. Phase-locked loop 24 and frequency divider 20 are part of a switch control of implantable medical device 10.

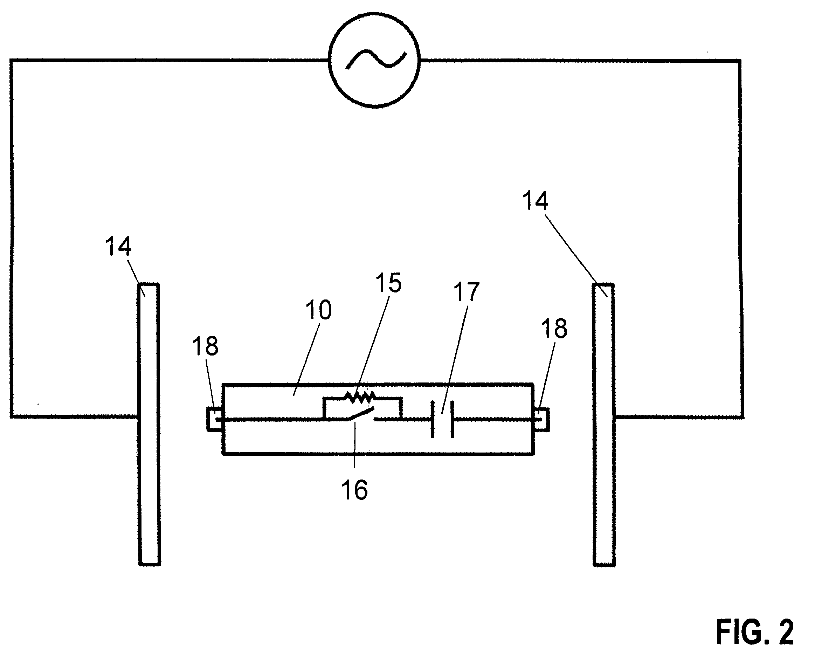

The field induced between the surface electrodes 14 is sensed by the implantable medical device 10. The implantable medical device 10 locks in the frequency of the electric field using the phase-locked loop 24. Once the implantable medical device 10 is locked on to the frequency of the external device's induced field, it can activate the switch 16 between the two electrodes 18 that are in the field (see Figs. 2 and 4) in synch with the frequency of the electric field.

In more detail: The implantable medical device 10 receives the imposed oscillatory electric signal as input signal that is detected via the electrode 18 or across a resistor. Thus, the implantable medical device has an input sine signal that can be detected as an alternating voltage across electrodes 18 or across a resistor. This input sine signal is band-pass filtered by band-pass filter 26. A representation of that band-pass filtered signal is shown in Fig. 4 (a).

The band-pass filtered input sine signal is fed to the phase-locked loop (PLL) 24 that locks in the frequency of the input sine signal. Phase-locked loop 24 puts out a synchronized sine signal to a comparator 22 that converts the sine signal Fig. 4 (a) to a square signal Fig. 4 (b). The square signal thus generated is fed to frequency divider 20 that generates a clock signal for switching the switching means 16. The clock signal thus generated has a frequency corresponding to a fraction of the frequency of the oscillatory electric field wherein the fraction is determined by a frequency division factor applied by frequency divider 20. The clock signal frequency is preferably in a range of 0.1 to 50 kHz, such as a range of 1 to 20 kHz, more preferably in a range of 4 to 10 kHz, most preferably the clock signal frequency is 8 kHz.

The actual switching of switching means 16 further depends on data that are to be transmitted from the implantable medical device 10 to the external device 12. The data to be transmitted is coded and the code determines the actual sequence of switching of switching means 16.

Frequency divider 20 can be realized as a flip-flop counter. Switching means 16 shall have a small on-resistance. The latter aspect leads to change of impedance, depending on whether switching means 16 are opened or closed.

Such change of impedance can be sensed by external device 12.

Data transmission from the implantable medical device 10 to the external device 12 thus can be summarized as follows:
Apply signal (oscillatory electric field) → propagate in body → switch on/off switching means 16 in implantable device 10 → impedance change of body → detect change by external device 12.

The switch control of the implantable medical device 10 thus receives an input sine signal by detecting a voltage across electrodes 18 or across a resistor. The switch control of the implantable medical device 10 according to the embodiment of Fig. 4 comprises band-pass filter 26, phase-locked loop 24 to lock in the frequency of the input sine signal, comparator 22 to convert the sine signal to a square signal, a flip-flop counter that acts as frequency divider 20 that control switching means 16. Switching means 16 has a small on-resistance.

The impedance changes caused by the implantable medical device 10 shown in Fig. 2 and 4 can be detected by the external device 12. As shown in Fig. 5, the external device 12 essentially comprises a lock-in amplifier 30 that generates an output signal (signal of Fig. 5 (c)) that represents the signal transmitted by implantable medical device 10 by way of impedance changes. Lock-in amplifier 30 uses the signal imposed on a body by means of surface electrodes 14 as a reference signal. For this purpose, a network of resistors 32 is provided that causes a voltage drop representing the signal (the oscillatory electric field) imposed on a body via surface electrodes 14.

This signal is amplified by pre-amplifier 34 of lock-in amplifier 30.

The amplified signal sensed via sensing means 43, i.e. surface electrodes 14 and the resistor network 32 is fed to an AM demodulator comprising phase-sensitive detector 36 and further fed to a low-pass filter 38, as depicted in Fig. 5. The amplified input signal sensed via surface electrodes 14 and the resistor network 32 is represented as signal (a) in Fig. 5 (a). The output signal of the phase-sensitive detector 36 is depicted as signal (b) in Fig. 5 (b). The low-pass filtered output signal of lock-in amplifier 30 is depicted in Fig. 5 (c) as signal (c). Signal (c) corresponds to the signal generated by implantable medical device 10 and thus represents data to be transmitted from implantable medical device 10 to external device 12. This signal can be analog-to-digital converted and stored. Block 40 in Fig. 5 represents an analog-to-digital converter (ADC) 41, a memory for data storage and a display of external device 12.

For the detection of impedance changes of the body caused by the implantable medical device 10, the external device 12 thus comprises a lock-in amplifier 30 that is configured to use the input as reference signal, comprising an AM demodulator which in turn comprises a precision rectifier and a low-pass filter 38. The low-pass filtered signal is fed to an analog-to-digital converter 41.

The communication from implantable medical device 10 to external device 12 thus can be understood as follows.

As the implantable medical device 10 alternatively shorts and opens the connection between the electrodes 18, the impedance between the external device electrodes 14 is slightly changed or modulated. The external device 12 can sense the change in impedance by measuring how much voltage or current is imparted on the electrodes 14 to create the oscillator electric field between the external device electrodes 14.

The external device 12 does this using a lock-in amplifier 30 that is synchronized to the electric field frequency and phase. As the implantable medical device 10 modulates the impedance between the external device electrodes 14, the external device 12 integrates the changing current or voltage. The integration allows a very small change in sourced voltage or current to be detected using amplitude modulation.

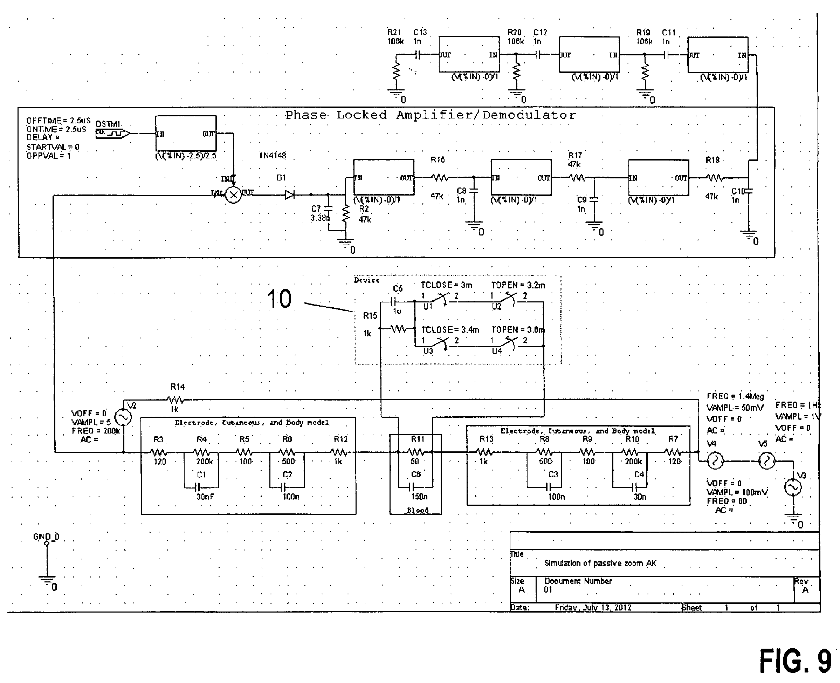

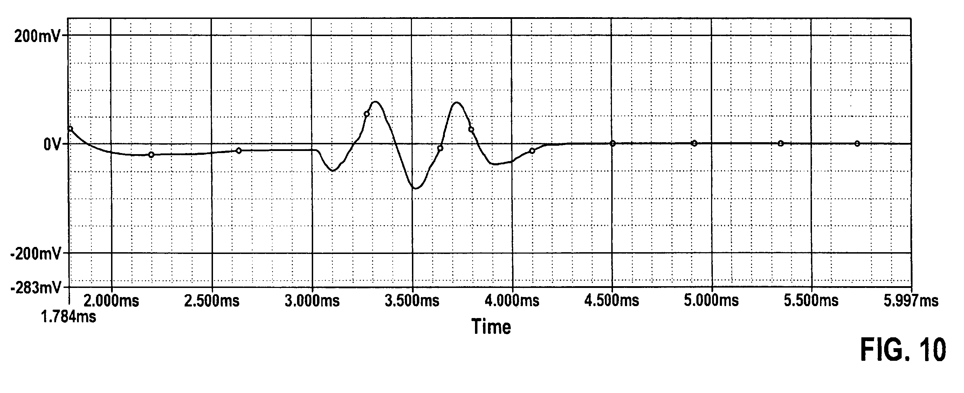

Communication can occur at approximately 1/10 to 1/200th of the modulation frequency of the oscillatory electric field. This allows for ∼10-100 cycles of the electric field to be integrated to determine the imparted current or voltage. A model and simulation of the embodiment is shown in Figs. 9 and 10.

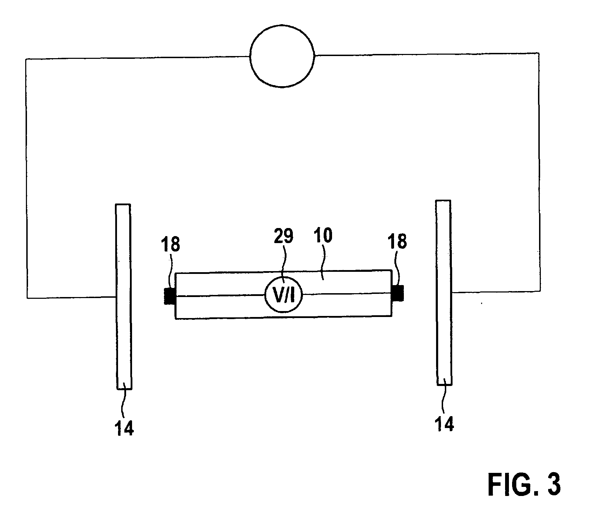

In an alternative embodiment shown in Fig. 3, a small electric field is generated by the implanted device 10 in place of passive resistance changes. The alternative embodiment of the implantable medical device 10 refers to circuitry that is located between the two surface electrodes 14 of the external device 12. The surface electrodes 14 of the external device 12 impart an oscillatory electric field on the body that encompasses the implantable medical device 10. The implantable medical device 10 monitors this field and a field generating means 29 generates a small electric field which is phase-synchronized with the field of the external device 12 imposed on body tissue surrounding the implantable medical device 10 in order to facilitate reception of this small field by the external device 12 using a lock-in amplifier 30 (see Fig. 5). The implantable medical device 10 according to this embodiment can comprise field generation control means that are operatively connected to the field generating means 29 and are configured to control the field generating means 29 in response to an oscillatory electric field imposed on a conductive medium surrounding the implantable medical device 10 when the implantable medical device 10 is in its implanted state or in a package. The small changes in the electric field are sensed by the external device 12 as changes in voltage or current. The changes in voltage or current are demodulated to form a communications scheme from the implantable medical device 10 to the external device 12.

In another embodiment, the implanted device 10 switches its impedance or small electric field at every other crest of the external device's applied electric field for ∼10-100 cycles, called a chirp, in order to represent a mark of the communications scheme.

A communication from external device 12 to implantable medical device 10 can be done as follows:
The imposed oscillatory electric field has a fundamental frequency that the implantable medical device 10 uses to lock onto. The fundamental frequency is also used as a carrier frequency to send modulated data to the implantable medical device 10. The external device 12 modulates data, using frequency modulation or amplitude modulation, on top of the carrier imparted electric field. The implantable medical device 10 decodes the modulated data sensed through the electrodes 18.

The present embodiment does not require the implanted medical device 10 to actively transmit data using its own power in the case of impedance modulation embodiment. It simply modulates a field imparted on it by an external device 12. In the alternative embodiment where the implantable medical device 10 does emit a low amplitude electric field, the phase-locked property that it maintains allows for lower amplitude to be used for transmission to the external device 12 than if simple band-limited transmission schemes were used. In both embodiments, the resulting drain on the implantable medical device's battery is extremely small. Because of the small power consumption, it is possible to transmit more data to the external device 12.

The problem of saturation of input amplifiers is apparent for impedance measurements and capture control. According to the prior art, this problem is solved as follows. After pacing pulses are delivered to a heart by the implantable medical device 10 the excess energy deposited on a DC blocking capacitor of the pace/sense circuitry 46 of the implantable medical device 10 must be removed. The DC blocking capacitor is shorted across the electrodes 18 to remove the excess voltage, which causes the input amplifier to saturate and which prevents sensing with the implantable medical device 10.

Figures 6 and 7 present embodiments of an implantable medical device 10 that allows communication with the external device 12 while sensing. Conductive intra-body communication uses a switch 16 across a tip electrode 18 and a ring electrode 18' of an implantable medical device 10 to alter the local electrical field to transmit data to the external device 12. This switching can cause large transient voltages to appear at the input amplifiers in a sense/pace circuitry 46 of the implantable medical device 10. The transient voltage appears because of DC offsets at the device electrodes due to electrode electrolyte interface. The large transient voltage can prevent sensing for several milliseconds thereby preventing sensing while communication with the external device 12 is ongoing. Electrically connecting a capacitor 17 in series with the data transmission switch 15 allows maintenance the offset voltage and thereby reduction of the transient voltage produced by switching, such that sensing can be maintained.

Figure 6 shows an electric circuit diagram of a part of an embodiment of an implantable medical device 10 in an implanted state with a conductive intra body communication switch 16, a tip electrode 18 and a ring electrode 18' (sense/pace circuitry 46 part is not shown in detail and represented by a block 46 connected via connection points).

An electrical double layer 42 (Helmholtz layer) forms on the surface of the ring 18' and tip electrodes 18 due to electrolytes present in the blood interacting with the electrical potential at the electrodes 18 and 18'. The electrical double layer 42 leads to a slight half-cell potential, which is represented as a DC voltage source for the implantable medical device 10 in the electric circuit diagram.

A capacitor 17 is electrically connected in series to the switch 16 to maintain the DC voltage on the electrodes 18, when the switch 16 is closed. When the switch shorts the electrodes 18 together, a high frequency carrier signal is shorted through the capacitor 17, and the local electric field is altered due to an instantaneous change in the electrical potential dependent on the tissue impedance, which effects communication. The DC offset voltage on the electrodes 18 is not affected and maintained by the capacitor 17 if the capacitor 17 has low impedance to high frequency, e.g., 1 Ohm and high impedance to DC and low frequency, preferably larger than 2500 Ohms.

An additional resistor 15 may be electrically connected in parallel with the switch 16 to maintain DC voltage on the capacitor 17 when communication is not active. The additional resistor 15 is optional. An impedance 44 electrically connects the electrical double layer 42 with the tip electrode 18 and closes the part of the electrical circuit of the implantable medical device 10 represented in Fig. 6.

Figure 7 shows the best contemplated mode for implementing a communication and sensing circuitry for simultaneous sensing and data communication of an implantable medical device 10 with an external device 12. The implementation of the switch circuit of the implantable medical device 10 is similar to the implementation of Fig. 6, but utilizes a low-pass filter at an electrogram amplifier front-end of the sense/pace circuitry 46 of the implantable medical device 10. A filter capacitor 17 is electrically connected in series to the switch 16 and a filter resistor 15 is electrically connected in parallel to the switch 16. The filter capacitor 17 acts to maintain the DC voltage on the electrodes 18 and 18' because it is continually charged by the filter resistor 15 bypassing the switch 16, when the switch 16 is open. The filter resistor 15, with preferably about 39 kOhm and filter capacitor 17, with preferably about 33 nF, act as a low-pass filter with a cutoff frequency of preferably about 124 Hz. The filter is configured to prevent high frequency noise from entering the electrogram amplifier. When the switch 16 is closed, the filter resistor 15 is shorted to low impedance and most of the input voltage falls across the filter capacitor 17. The cutoff frequency (filter corner) is shifted to a higher frequency of about 300 kHz depending on the switch impedance. The high frequency carrier signal is shunted through the filter capacitor 17 to the other electrode 18. The switch 16 in this configuration allows sensing of low frequency electrocardiograms while shunting high frequency carrier signals from one electrode, e.g., the tip electrode 18 to the other electrode, e.g., the ring electrode 18'. This shunting of the high frequency alters the local electric field around the implantable medical device 10 allowing communication.

Charge injection by the switch 16 may cause slight noise on the electrogram sensing. There is a trade-off between charge injection and switch impedance. If charge injection by the switch 16 is high, the charge will be deposited on the filter capacitor 17 and the electrodes 18 and cause switching artifact. The electrogram can nonetheless be acquired but high frequency noise may be present. Switch impedance affects the amplitude of the reflected signal to the external device 12. The higher the impedance the lower the reflected signal. Therefore the ideal switch 12 has low charge injection and low impedance.
In a further embodiment, a plurality of filter resistors 15 may be used. In this case filter resistor 15 is formed by a plurality of resistors that are connected by additional switches in parallel to switch 16. In this embodiment different values of filter resistor 15 can be used.

For example, if two different values for resistor 15 are used, two bit of information can be transmitted in parallel, e.g. switch 16 open and low value of resistor 15 represent 00, switch 16 open and high value of resistor 15 represents 01, switch 16 closed and low value of resistor 15 represents 10 and switch closed and high value of resistor 15 represents 11.

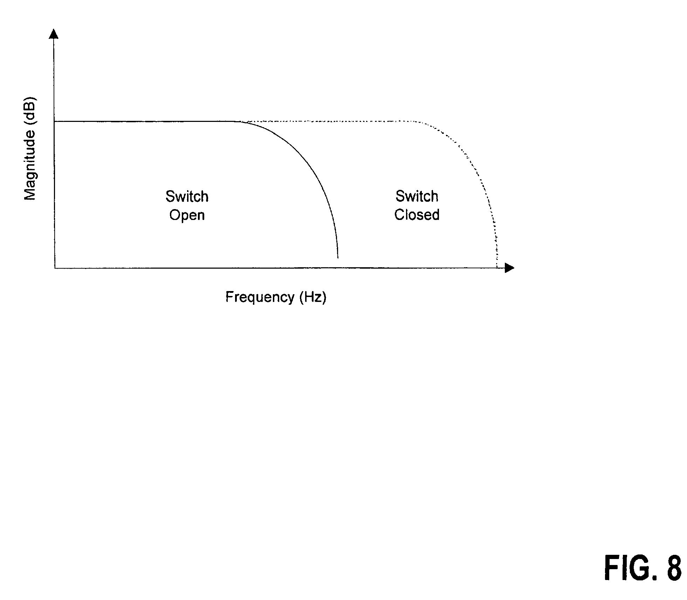

In Fig. 8 a diagram of the magnitude in dB in dependence of the frequency in Hz illustrates the change of the frequency response due to switching the switch 16. The low-pass filter cutoff frequency (corner) changes during transmission, when the switch 16 is changed from an open to a closed position. Preferably the low-pass filter cutoff frequency is in the range of 100 to 200 Hz, most preferably about 124 Hz in an open state. The low-pass filter is used to prevent high frequency noise from entering the amplifier. Switching the switch 16 shifts the cutoff frequency (corner) to a higher frequency in dependence of the switch impedance, e.g., to about 300 kHz in a closed state of the switch 16.

Figures 9 and 10 illustrate a simulation of passive impedance-based data communication.

Standard electrode-electrolyte-body impedance models and blood impedance models were combined with a lock-in demodulator in a model of the passive impedance implementation of the invention. Each simulation component is labeled, with the 'device' modeled in a box labeled with "10" with access impedance and four closing switches to simulate the communication of two data bits to the external receiver/demodulator. Environmental and breathing noise is induced in the model with voltage sources V3, V4, and V5 simulating line noise, rf noise, and breathing, body changing impedance noise, respectively.

The simulation signal at output filter resistor R21 is shown as the trace in Fig. 10. The impedance changes are at a detectable level (showing 2 bits) beyond the background noise which has been filtered out due to the lock-in receiver system. The slope to the left of this plot shows the final settling time of the filters.

## Claims

1. Implantable device (10) comprising data communication means, said data communication means including means (21) for altering an oscillatory electric field imposed on a conductive medium surrounding the implantable device (10), wherein the oscillatory electric field has a frequency within a range of approximately 50 kHz to approximately 1 MHz, wherein the means (21) for altering an oscillatory electric field are configured to modulate an impedance of the conductive medium surrounding the implantable device (10) when the implantable device (10) is within an oscillatory electric field, further comprising switching means (16) and at least two electrodes (18, 18') that are connected to the switching means (16) so they can be electrically connected or disconnected, respectively, by the switching means (16), wherein the at least two electrodes (18) are configured and arranged to cause a change of impedance of the conductive medium and the electrodes (18, 18') are connected or disconnected, respectively, by means of the switching means (16), wherein the switching means (16) alternates the impedance between the at least two electrodes such that the oscillatory electric field is modulated and low frequency signals are not modulated.

2. Implantable device (10) according to claim 1, wherein the implantable device (10) comprises a capacitor (17) electrically connected in series with the switching means (16) and between the two electrodes (18, 18') and a resistor (15) electrically connected in parallel with the switching means (16) of the implantable device (10).

3. Implantable device (10) according to at least one of the claims 1 or 2, wherein the switching means (16) are connected to a switch control that is configured to sense an oscillatory electric field imposed on a conductive medium surrounding the implantable device (10).

4. Implantable device (10) according to claim 3, wherein the switch control comprises a phase-locked loop (PLL) (24) and a frequency divider (20), wherein the phase-locked loop (24) is configured to lock in a frequency of an oscillatory electric field imposed on a conductive medium surrounding the implantable device (10), and wherein the frequency divider (20) is connected to the phase-locked loop (24) and is configured to divide a frequency signal put out by the phase-locked loop (24).

5. Implantable device (10) according to claims 1 and 3, wherein the switch control is connected to the at least two electrodes (18, 18') and is configured to sense an oscillatory electric field imposed on a conductive medium via the at least two electrodes (18, 18').

6. Implantable device (10) according to claim 4, wherein the switch control comprises a band-pass filter (26), wherein the band-pass filter (26) filters a signal fed to the phase-locked loop (24).

7. Implantable device (10) according to claim 1, wherein the means (29) for altering an oscillatory electric field comprise field generating means (29) for generating an oscillatory electric field that is phase-synchronized with an oscillatory electric field imposed on a conductive medium surrounding the implantable device (10).

8. Implantable device (10) according to claim 7, further comprising field generation control means that are operatively connected to the field generating means (29) and are configured to control the field generating means (29) in response to an oscillatory electric field imposed on a conductive medium surrounding the implantable device (10).

9. Data communication system including an implantable device (10) according to at least one of the claims 1 to 8 and further comprising an external device (12) that comprises or can be connected to at least two surface electrodes (14), wherein the external device (12) further comprises external field generating means that are configured to generate an oscillatory electric field to be imposed on a body via the at least two surface electrodes (14), wherein the external device (12) further comprises means (43) for sensing alterations of impedance and/or an oscillatory electric field generated by the implantable device (10).

10. Data communication system according to claim 9, wherein the external device (12) comprises a lock-in amplifier (30), an AM demodulator for demodulating amplitude-modulated signals and an analog-to-digital converter (41), wherein the analog-to-digital converter (41) is operatively connected to the AM demodulator and the lock-in amplifer (30), and wherein the analog-to-digital converter (41) puts out a signal that represents a signal transmitted by the implantable device (10).

11. Method of communicating data from an implantable device (10) to an external device (12), said method comprising:
- imposing an oscillatory electric field on a conductive medium encompassing an implantable device (10),
- altering the oscillatory electric field by means of the implantable device (10)
- by inducing an alternating change of impedance by means of the implantable device (10)
- or by generating a small oscillatory electric field by means of the implantable device (10),
- sensing the change of impedance or the small oscillatory electric field generated by the implantable device (10), respectively, by means of an external device (12) having or being connected to at least two surface electrodes (14), wherein the oscillatory electric field has a frequency within a range of approximately 50 kHz to approximately 1 MHz,
- wherein the step of altering the oscillatory electric field by means of the implantable device (10) is performed by means of at least two electrodes (18, 18') operatively connected or being part of the implantable device (10) and switching means (16) operatively connected to the at least two electrodes (18, 18'), wherein the switching means (16) connect and disconnect the at least two electrodes (18, 18') in an alternating manner according to a code representing data to be transmitted from the implantable device (10) to the external device (12), said alternating connecting and disconnecting causing a detectable change of impedance for the imposed oscillatory electric field.

12. Method according to claim 11, wherein the step of sensing the change of impedance comprises sensing a current or voltage over surface electrodes (14) and integrating a sensed voltage or current over a number of cycles of the oscillatory electric field, said number of cycles corresponding to a frequency division factor of a frequency divider (20) of the implantable device (10).

13. Method according to claim 11, wherein the imposed oscillatory electric field is sensed by means of the implantable medical device after imposing an oscillatory electric field on a conductive medium encompassing an implantable medical device.

14. A communication system comprising:
an external communication device comprising at least two surface electrodes connected to an external communication module;
an internal implantable communication device comprising at least two electrodes connected to an internal communication module;
wherein said external communication module is configured to impart an oscillatory electric field via the at least two surface electrodes across said internal communication device and to detect an impedance change surrounding said internal communication device caused by the internal communication module, and
wherein the oscillatory electric field has a frequency within a range of approximately 50 kHz to approximately 1 MHz.

## Patentansprüche

1. Implantierbares Produkt (10) mit
Datenübertragungsmitteln, wobei die Datenübertragungsmittel Mittel (21) zum Ändern eines schwingenden elektrischen Felds aufweisen, das einem das implantierbare Produkt (10) umgebenden leitfähigen Medium aufgeprägt wird, wobei
- das schwingende elektrische Feld eine Frequenz in einem Bereich von ungefähr 50 kHz bis ungefähr 1 MHz aufweist, wobei die Mittel (21) zum Ändern eines schwingenden elektrischen Felds so ausgelegt sind, dass sie eine Impedanz des leitfähigen Mediums, das das implantierbare Produkt (10) umgibt, modulieren, wenn sich das implantierbare Produkt (10) innerhalb eines schwingenden elektrischen Felds befindet,
ferner umfassend ein Schaltmittel (16) und mindestens zwei Elektroden (18, 18'), die mit dem Schaltmittel (16) verbunden sind, sodass sie von dem Schaltmittel (16) elektrisch verbunden beziehungsweise getrennt werden können, wobei
- die mindestens zwei Elektroden (18) so ausgelegt und angeordnet sind, dass sie eine Impedanzänderung des leitfähigen Mediums bewirken, und die Elektroden (18, 18') von dem Schaltmittel (16) verbunden beziehungsweise getrennt werden, wobei das Schaltmittel (16) die Impedanz zwischen den mindestens zwei Elektroden derart ändert, dass das schwingende elektrische Feld moduliert wird und niederfrequente Signale nicht moduliert werden.

2. Implantierbares Produkt (10) nach Anspruch 1, wobei das implantierbare Produkt (10) einen Kondensator (17) umfasst, der elektrisch in Reihe mit dem Schaltmittel (16) und zwischen die beiden Elektroden (18, 18') geschaltet ist, sowie einen Widerstand (15), der elektrisch parallel mit dem Schaltmittel (16) des implantierbaren Produkts (10) geschaltet ist.

3. Implantierbares Produkt (10) nach mindestens einem der Ansprüche 1 oder 2, wobei die Schaltmittel (16) mit einer Schaltsteuerung verbunden sind, die so ausgelegt ist, dass sie ein schwingendes elektrisches Feld erfasst, das einem leitfähigen Medium aufgeprägt wird, das das implantierbare Produkt (10) umgibt.

4. Implantierbares Produkt (10) nach Anspruch 3, wobei die Schaltsteuerung eine Phasenregelschleife (PLL) (24) und einen Frequenzteiler (20) umfasst, wobei die Phasenregelschleife (24) so ausgelegt ist, dass sie bei einer Frequenz eines schwingenden elektrischen Felds einrastet, das einem leitfähigen Medium aufgeprägt wird, das das implantierbare Produkt (10) umgibt, und wobei der Frequenzteiler (20) mit der Phasenregelschleife (24) verbunden und so ausgelegt ist, dass er ein Frequenzsignal teilt, das die Phasenregelschleife (24) ausgibt.

5. Implantierbares Produkt (10) nach Anspruch 1 und 3, wobei die Schaltsteuerung mit den mindestens zwei Elektroden (18, 18') verbunden und so ausgelegt ist, dass sie ein schwingendes elektrisches Feld erfasst, das über die mindestens zwei Elektroden (18, 18') einem leitfähigen Medium aufgeprägt wird.

6. Implantierbares Produkt (10) nach Anspruch 4, wobei die Schaltsteuerung ein Bandpassfilter (26) umfasst, wobei das Bandpassfilter (26) ein der Phasenregelschleife (24) zugeführtes Signal filtert.

7. Implantierbares Produkt (10) nach Anspruch 1, wobei die Mittel (29) zum Ändern eines schwingenden elektrischen Felds Felderzeugungsmittel (29) zum Erzeugen eines schwingenden elektrischen Felds umfassen, das zu einem schwingenden elektrischen Feld phasensynchronisiert ist, das einem leitfähigen Medium aufgeprägt wird, das das implantierbare Produkt (10) umgibt.

8. Implantierbares Produkt (10) nach Anspruch 7, ferner umfassend Felderzeugungssteuerungsmittel, die funktionsmäßig mit den Felderzeugungsmitteln (29) verbunden und so ausgelegt sind, dass sie die Felderzeugungsmittel (29) als Reaktion auf ein schwingendes elektrisches Feld steuern, das einem leitfähigen Medium aufgeprägt wird, das das implantierbare Produkt (10) umgibt.

9. Datenübertragungssystem, aufweisend ein implantierbares Produkt (10) nach mindestens einem der Ansprüche 1 bis 8 und ferner umfassend eine externe Einrichtung (12), die mindestens zwei Oberflächenelektroden (14) umfasst oder damit verbunden sein kann, wobei die externe Einrichtung (12) ferner externe Felderzeugungsmittel umfasst, die so ausgelegt sind, dass sie ein schwingendes elektrisches Feld erzeugen, das über die mindestens zwei Oberflächenelektroden (14) einem Körper aufgeprägt werden soll, wobei die externe Einrichtung (12) ferner Mittel (43) zum Erfassen von Impedanzänderungen und/oder eines schwingenden elektrischen Felds umfasst, das von dem implantierbaren Produkt (10) erzeugt wird.

10. Datenübertragungssystem nach Anspruch 9, wobei die externe Einrichtung (12) einen Lock-in-Verstärker (30), einen AM-Demodulator zum Demodulieren von amplitudenmodulierten Signalen und einen Analog-Digital-Wandler (41) umfasst, wobei der Analog-Digital-Wandler (41) funktionsmäßig mit dem AM-Demodulator und dem Lock-in-Verstärker (30) verbunden ist und wobei der Analog-Digital-Wandler (41) ein Signal ausgibt, das ein Signal darstellt, das das implantierbare Produkt (10) sendet.

11. Verfahren zum Übertragen von Daten von einem implantierbaren Produkt (10) an eine externe Einrichtung (12), wobei das Verfahren Folgendes umfasst:
- Aufprägen eines schwingenden elektrischen Felds auf ein leitfähiges Medium, das ein implantierbares Produkt (10) umgibt,
- Ändern des schwingenden elektrischen Felds mit dem implantierbaren Produkt (10),
- indem mit dem implantierbaren Produkt (10) eine wechselnde Änderung der Impedanz bewirkt wird
- oder indem mit dem implantierbaren Produkt (10) ein kleines schwingendes elektrisches Feld erzeugt wird,
- Erfassen der Impedanzänderung beziehungsweise des von dem implantierbaren Produkt (10) erzeugten kleinen schwingenden elektrischen Felds mit einer externen Einrichtung (12), die mindestens zwei Oberflächenelektroden (14) aufweist oder damit verbunden ist, wobei das schwingende elektrische Feld eine Frequenz in einem Bereich von ungefähr 50 kHz bis ungefähr 1 MHz aufweist,
- wobei der Schritt des Änderns des schwingenden elektrischen Felds mit dem implantierbaren Produkt (10) mithilfe von mindestens zwei Elektroden (18, 18'), die funktionsmäßig mit dem implantierbaren Produkt (10) verbunden oder Bestandteil davon sind, und Schaltmitteln (16) durchgeführt wird, die funktionsmäßig mit den mindestens zwei Elektroden (18, 18') verbunden sind, wobei die Schaltmittel (16) die mindestens zwei Elektroden (18, 18') abwechselnd entsprechend einem Code verbinden und trennen, der Daten darstellt, die von dem implantierbaren Produkt (10) an die externe Einrichtung (12) übertragen werden sollen, wobei das abwechselnde Verbinden und Trennen eine nachweisbare Impedanzänderung für das aufgeprägte schwingende elektrische Feld bewirkt.

12. Verfahren nach Anspruch 11, wobei der Schritt des Erfassens der Impedanzänderung ein Erfassen eines Stroms oder einer Spannung über Oberflächenelektroden (14) und ein Integrieren einer erfassten Spannung oder eines erfassten Stroms über mehrere Zyklen des schwingenden elektrischen Felds umfasst, wobei die Zyklenzahl einem Frequenzteilungsfaktor eines Frequenzteilers (20) des implantierbaren Produkts (10) entspricht.

13. Verfahren nach Anspruch 11, wobei das aufgeprägte schwingende elektrische Feld mit dem implantierbaren Medizinprodukt erfasst wird, nachdem ein schwingendes elektrisches Feld einem leitfähigen Medium aufgeprägt wurde, das ein implantierbares Medizinprodukt umgibt.

14. Übertragungssystem, umfassend:
- eine externe Übertragungseinrichtung, die mindestens zwei Oberflächenelektroden umfasst, die mit einem externen Übertragungsmodul verbunden sind;
- eine interne implantierbare Übertragungseinrichtung, die mindestens zwei Elektroden umfasst, die mit einem internen Übertragungsmodul verbunden sind;
- wobei das externe Übertragungsmodul so ausgelegt ist, dass es über die mindestens zwei Oberflächenelektroden an die interne Übertragungseinrichtung ein schwingendes elektrisches Feld überträgt und eine die interne Übertragungseinrichtung umgebende Impedanzänderung erkennt, die von dem internen Übertragungsmodul verursacht wird, und
- wobei das schwingende elektrische Feld eine Frequenz in einem Bereich von ungefähr 50 kHz bis ungefähr 1 MHz aufweist.

## Revendications

1. Dispositif implantable (10) comprenant
des moyens de communication de données, lesdits moyens de communication de données incluant des moyens (21) pour modifier un champ électrique oscillatoire imposé sur un milieu conducteur entourant le dispositif implantable (10), où
- le champ électrique oscillatoire a une fréquence dans une plage d'approximativement 50 kHz à approximativement 1 MHz, où les moyens (21) pour modifier un champ électrique oscillatoire sont configurés pour moduler une impédance du milieu conducteur entourant le dispositif implantable (10) lorsque le dispositif implantable (10) est dans un champ électrique oscillatoire,
comprenant en outre des moyens de commutation (16) et au moins deux électrodes (18, 18') qui sont connectées aux moyens de commutation (16) de sorte qu'elles puissent être connectées ou déconnectées électriquement, respectivement, par les moyens de commutations (16), où
- les au moins deux électrodes (18) sont configurées et agencées pour provoquer une variation d'impédance du milieu conducteur et les électrodes (18, 18') sont connectées ou déconnectées, respectivement, au moyen des moyens de commutation (16), où les moyens de commutation (16) font alterner l'impédance entre les au moins deux électrodes de sorte que le champ électrique oscillatoire est modulé et des signaux de faible fréquence ne sont pas modulés.

2. Dispositif implantable (10) selon la revendication 1, où le dispositif implantable (10) comprend un condensateur (17) connecté électriquement en série avec les moyens de commutation (16) et entre les deux électrodes (18, 18') et une résistance (15) électriquement connectée en parallèle avec les moyens de commutation (16) du dispositif implantable (10).

3. Dispositif implantable (10) selon au moins l'une des revendications 1 ou 2, où les moyens de commutation (16) sont connectés à une commande d'interrupteur qui est configurée pour percevoir un champ électrique oscillatoire imposé à un milieu conducteur entourant le dispositif implantable (10).

4. Dispositif implantable (10) selon la revendication 3, où la commande d'interrupteur comprend une boucle à verrouillage de phase (PLL) (24) et un diviseur de fréquence (20), où la boucle à verrouillage de phase (24) est configurée pour verrouiller une fréquence d'un champ électrique oscillatoire imposé à un milieu conducteur entourant le dispositif implantable (10), et où le diviseur de fréquence (20) est connecté à la boucle de verrouillage de phase (24) et est configuré pour diviser un signal de fréquence délivré par la boucle de verrouillage de phase (24).

5. Dispositif implantable (10) selon les revendications 1 et 3, où la commande d'interrupteur est connectée aux au moins deux électrodes (18, 18') et est configurée pour percevoir un champ électrique oscillatoire imposé à un milieu conducteur via les au moins deux électrodes (18, 18').

6. Dispositif implantable (10) selon la revendication 4, où la commande d'interrupteur comprend un filtre passe bande (26), où le filtre passe-bande (26) filtre un signal alimentant la boucle de verrouillage de phase (24).

7. Dispositif implantable (10) selon la revendication 1, où les moyens (29) pour modifier un champ électrique oscillatoire comprennent des moyens de génération de champ (29) pour générer un champ électrique oscillatoire qui est synchronisé en phase avec un champ électrique oscillatoire imposé sur un milieu conducteur entourant le dispositif implantable (10).

8. Dispositif implantable (10) selon la revendication 7, comprenant en outre des moyens de commande de génération de champ qui sont connectés de manière opérationnelle aux moyens de génération de champ (29) et sont configurés pour commander les moyens de génération de champ (29) en réponse à un champ électrique oscillatoire imposé à un milieu conducteur entourant le dispositif implantable (10).

9. Système de communication de données incluant un dispositif implantable (10) selon au moins l'une des revendications 1 à 8, et comprenant en outre un dispositif externe (12) qui comprend, ou peut être connecté à au moins deux électrodes de surface (14), où le dispositif externe (12) comprend en outre des moyens de génération d'un champ externe qui sont configurés pour générer un champ électrique oscillatoire devant être imposé à un corps via les au moins deux électrodes de surface (14), où le dispositif externe (12) comprend en outre des moyens (43) pour percevoir des altérations de l'impédance et/ou du champ électrique oscillatoire générées par le dispositif implantable (10).

10. Système de communication de données selon la revendication 9, où le dispositif externe (12) comprend un amplificateur synchrone (30), un démodulateur d'amplitude AM pour démoduler les signaux modulés en amplitude et un convertisseur analogique-numérique (41), où le convertisseur analogique-numérique (41) est connecté de manière opérationnelle au démodulateur d'amplitude AM et à l'amplificateur synchrone (30), et où le convertisseur analogique-numérique (41) émet un signal qui représente un signal transmis par le dispositif implantable (10).

11. Procédé de communication de données provenant d'un dispositif implantable (10) vers un dispositif externe (12), ledit procédé comprenant :
- l'imposition d'un champ électrique oscillatoire sur un milieu conducteur englobant un dispositif implantable (10),
- la modification du champ électrique oscillatoire au moyen du dispositif implantable (10)
- en induisant une variation alternative d'impédance au moyen du dispositif implantable (10)
- ou en générant un petit champ électrique oscillatoire au moyen du dispositif implantable (10),
- la perception d'une variation d'impédance ou du petit champ électrique oscillatoire généré par le dispositif implantable (10), respectivement, au moyen d'un dispositif externe (12) ayant, ou étant connecté aux au moins deux électrodes de surface (14), où le champ électrique oscillatoire a une fréquence dans une plage d'approximativement 50 kHz à approximativement 1 MHz,
- où l'étape de modification du champ électrique oscillatoire au moyen du dispositif implantable (10) est effectuée au moyen d'au moins deux électrodes (18, 18') connectées de manière opérationnelle ou faisant partie du dispositif implantable (10) et des moyens de commutation (16) connectés de manière opérationnelle aux au moins deux électrodes (18, 18'), où les moyens de commutation (16) connectent et déconnectent les au moins deux électrodes (18, 18') d'une manière alternative selon une donnée représentant un code pour être transmis à partir du dispositif implantable (10) vers le dispositif externe (12), lesdites connexion et déconnexion alternatives provoquant une variation d'impédance détectable pour le champ électrique oscillatoire imposé.

12. Procédé selon la revendication 11, où l'étape de perception de la variation d'impédance comprend la perception d'un courant ou d'une tension sur les électrodes de surfaces (14) et l'intégration d'une tension ou d'un courant perçu(e) sur un nombre de cycles du champ électrique oscillatoire, ledit nombre de cycles correspondant à un facteur de division de fréquence d'un diviseur de fréquence (20) du dispositif implantable (10).

13. Procédé selon la revendication 11, où le champ électrique oscillatoire imposé est perçu au moyen du dispositif médical implantable après l'imposition d'un champ électrique oscillatoire sur un milieu conducteur englobant un dispositif médical implantable.

14. Système de communication comprenant :
- un dispositif de communication externe comprenant au moins deux électrodes de surface connectées à un module de communication externe ;
- un dispositif de communication interne implantable comprenant au moins deux électrodes connectées au module de communication interne ;
- où ledit module de communication externe est configuré pour communiquer un champ électrique oscillatoire via les au moins deux électrodes de surface à travers ledit dispositif de communication interne et pour détecter une variation d'impédance entourant ledit dispositif de communication interne provoquée par le module de communication interne, et
- où le champ électrique oscillatoire a une fréquence dans une plage d'approximativement 50 kHz à approximativement 1 MHz.
